# EUROPEAN PATENT APPLICATION

(11) **EP 2 075 327 A1**
(43) Date of publication of application: **01.07.2009**
(21) Application number: 07721549.9
(22) Date of filing: 25.06.2007
(51) Int. Cl.: C12N 1/04, C12N 1/20, C12P 7/24, C12R 1/01, C12R 1/465

(54) **A STREPTOMYCES STRAIN AND THE METHOD OF CONVERTING FERULIC ACID TO VANILLIN BY USING THE SAME**

(30) Priority: 20.10.2006 CN 200610117386
(71) Applicant: Shanghai Apple Flavor&Fragrance Co. Ltd., Jiading District Shanghai 201809 (CN); Shanghai Kaixin Biotech Co., Ltd, Jiading District, Shanghai 201809 (CN)
(72) Inventor: XU, Ping, Shanghai 201809 (CN); HUA, Dongliang, Shanghai 201809 (CN); LIN, Shan, Shanghai 201809 (CN); SONG, Lifu, Shanghai 201809 (CN); MA, Cuiqing, Shanghai 201809 (CN); ZHANG, Zhaobin, Shanghai 201809 (CN); ZENG, Yiyong, Shanghai 201809 (CN); DU, Yi, Shanghai 201809 (CN); CHEN, Hong, Shanghai 201809 (CN); GAN, Li, Shanghai 201809 (CN); WEI, Zhonghao, Shanghai 201809 (CN)
(74) Representative: Van Reet, Joseph
(86) International application number: PCT/CN2007/001976
(87) International publication number: WO 2008/049299

(57) **Abstract**

A method of vanillin production from ferulic acid with a high concentration by biotransformation using a Streptomyces sp. strain is claimed in this invention. This strain is named as Streptomyces sp. V-1, which has been deposited in China Center for Type Culture Collection on July 12, 2006 with the number of CCTCC M 206065. Using this strain, high concentration of vanillin fermentation broth is obtained from ferulic acid by biotransformation in GY biotransformation medium. With the addition of macroporous adsorbent resin DM11, the concentration of vanillin can be greatly improved. The advantage of this invention is less environmental pollution, high product concentration, less by-product, short processing cycle, low production cost, simple product extraction, clean production process, product environmental friendly, safe and reliable, which solves many difficulties in the vanillin production from botanical raw material extraction or chemical synthesis, and therefore it has good application prospect.

## Description

### Field of the invention

This invention belongs to the domain of biotechnology, and in detail especially relates to a method of vanillin production from ferulic acid by biotransformation using a Streptomyces sp. strain.

### Background of the invention

Vanillin has a molecular formula of C8H803 and molecular weight of 152.14. The appearance of vanillin is white, slight yellow crystal or powder, with a typical odor of Vanilla pods and slight sweet. In moist air vanillin is slowly oxidized to vanillic acid. It has a boiling point of 284 ∼ 285°C and a relative density of 1.056. Vanillin has a slight solubility in water but has a good solubility in organic solvent such as ethanol, ether, chloroform, acetic acid, carbon bisulfide and pyridine.

Vanillin is one of the most widely used flavors with the highest yield, which is named as "emperor of flavors". It is widely used in the food industry such as chocolate, ice cream, candy, cigarette, beverage and cosmetic. Vanillin not only can be used as flavor-adding agent and flavor-stabilizing agent, but also can be used ad antiseptic, keeping-fresh agent and antioxidant. In chemical industry, it is applied to prevent lubricating agent from foaming and used as whitening agent or active agent in zinc plating. Vanillin is also an important substrate for drug synthesis including L-dopa, L-methyldopa and narceine. It has a world market of 15,000 tons per year, which is growing steadily at 10%.

Today vanillin is extracted from botanic raw materials or is chemically synthesized. Vanillin is the main component of natural Vanilla, and has a concentration of 15 ∼ 20 g kg-1 in Vanilla pods. However, Vanilla can not be planted in large area because of man-made pollination and high work intensity. And vanillin production from Vanilla pods is only 2,000 tons per year and can not meet the demand of growing market for natural vanillin. Most vanillin in the market today is chemically synthesized from eugenol, lignin, glyoxylic acid, safrole or guaiacol. In China a nitrify method of Reimer-tieman reaction is mostly used to synthesize vanillin using guaiacol. This method has been washed out abroad because of the long-line, complexity of products extraction, low yield, serious environmental pollution and high material consumption. A synthetic method with simple equipments and high yield using guaiacol and glyoxylic acid is mostly used abroad. However, it is still in the initial laboratory phase and can not be industrially applied. In summary, though the chemical synthesis method has the characteristic of mature technique, high product purity and sufficient material supply, disadvantages such as serious environmental pollution, single aroma of product and easy impurity doping exist. Furthermore, the safety of synthesized vanillin is queried.

With the improvement of living conditions, demands for natural or green products increase. Contrasted to chemical synthesis, biological way has the advantages such as mild reaction condition, few byproducts, simple product extraction, clear production process, less environmental pollution and the safety of vanillin. Therefore, biological production of vanillin has attracted more and more attention. According to FDA legislation, only products obtained from plant and animal materials or obtained from microbial, enzymatic biotransformation can be identified as natural. It is found that many substrates can be transformed to vanillin under the catalysis of Bacillus, Streptomyces, Pseudomonas and Serratia. These substrates include ferulic acid, lignin, vanillic acid, eugenol and isoeugenol. Among them, ferulic acid and eugenol are mostly studied. However, vanillin concentration from eugenol is very low and is far away from industrial application.

Many microorganisms have been reported capable of transforming ferulic acid to vanillin, and most vanillin yields are below 10 g I-1. The two highest vanillin yields are obtained from Streptomyces setonii ATCC39116 (Rabenhorst 1996) and Amycolatopsis sp. HR167 (Muheim et al. 1999), in which the concentrations of vanillin are 12 g I-1 and 11.5 g I-1, respectively. Such high concentrations of vanillin have reached its crystallization point of 10 g I-1, and therefore it is close to industrial application. However, culture media used in the vanillin production applying these two strains are complex leading to high production cost and productions of many byproducts including vanillic acid, vanillyl alcohol and guaiacol, which make extraction process difficult.

### SUMMARY OF THE INVENTION

Aiming at the market demands for natural and green products and in order to solve the problem of low yield, too many byproducts and high production cost lying in the biological production process of vanillin, the present invention relates to a Streptomyces strain, named as Streptomyces sp. V-1, which has been deposited in China Center for Type Culture Collection on July 12, 2006 with the number of CCTCC M 206065. The characteristic of this strain is: aerial mycelium is brilliant yellow and substrate mycelium is slight yellow. Substrate mycelium has no septum and fragment. The strain can utilize glucose, xylose, fructose, sucrose. Spores are elliptical and the surface is smooth. The strain can not utilize glutin and no hydrogen sulfide is produced. Its 16S rDNA sequence has a similarity of 97 ∼ 98% to those of many Streptomyces strains.

A method of vanillin production from ferulic acid by biotransformation using a strain Streptomyces sp. V-1, wherein detailed steps are as follows:
1. Cultivation of slants: strain Streptomyces sp. V-1 is inoculumed to solid slants and statically cultivated at 30°C for 24 h.
2. Preparation of seed culture: slants obtained from step 1 are inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 10 ∼ 30 h.
4. Biotransformation: ferulic acid (5 ∼ 15 g I-1) is added to the culture obtained from step 3 and cultivated at 30°C, 120 rpm min-1 for 12 ∼ 24 h. In the fed-batch biotransformation of ferulic acid, macroporous adsorbent resin DM11 is added (2% ∼ 12%, wet weight/volume) after 12-h cultivation. When the concentration is below 2 g I-1, additional 5 g I-1 ferulic acid is added until the final concentration of ferulic acid reaches 45 g I-1. The total biotransformation time is 55 h.
Therein,
optimal cultivation time in step 3 is 18 ∼ 28 h,
optimal concentration of ferulic acid added in step 4 is 5 ∼ 15 g I-1,
optimal biotransformation time in step 4 is 10 ∼ 20 h,
optimal amount of macroporous adsorbent resin added is 5% ∼ 10%.

In the above method of vanillin production from ferulic acid by biotransformation using a Streptomyces sp. strain, the seed medium contains glucose 10 g I-1, yeast powder 5 g I-1, peptone 10 g I-1, malt extract 10 g I-1, sodium chloride 5 g I-1 (pH 7.2). The above medium is sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The above medium is sterilized at 115°C for 20 min.

With the method of vanillin production from ferulic acid by biotransformation using strain Streptomyces sp. V-1, advantages of this invention lie in: mild reaction conditions, less environmental pollution, high product concentration, less byproducts, short processing cycle, low production cost, simple product extraction and clean production process, environmental friendly and the safety of products. Therefore, this method solves many difficulties in the vanillin production process of botanical raw material extraction or chemical synthesis, and therefore has good application prospect.

### DISCRIPTION OF THE DRAWINGS

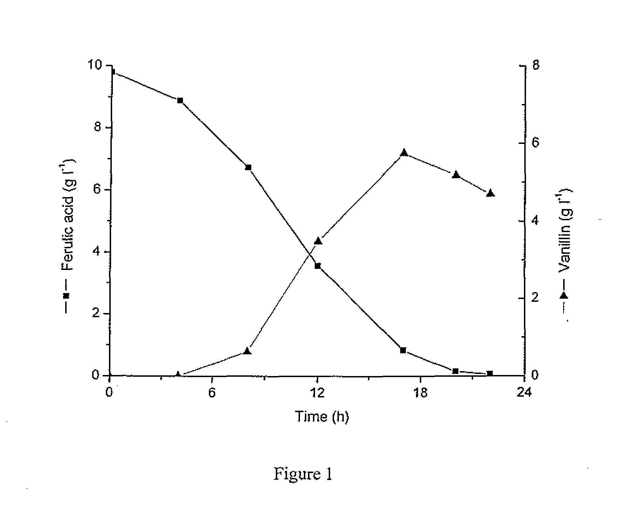

Figure 1 illustrates the time course of vanillin production from ferulic acid using strain Streptomyces sp. V-1.

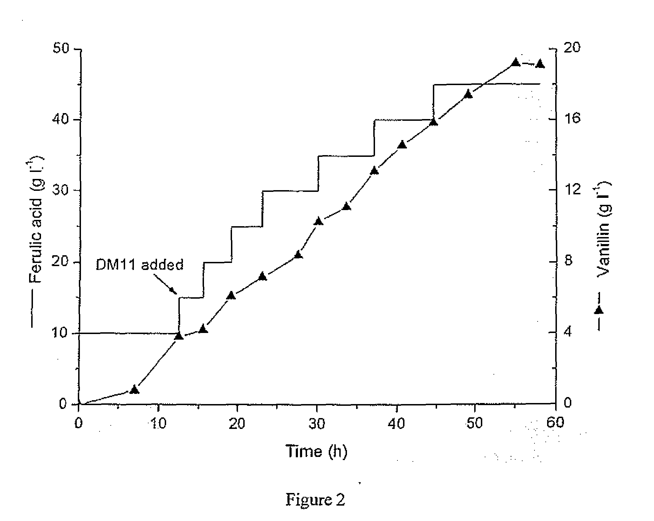

Figure 2 illustrates the time course of vanillin production from fed-batch biotransformation of ferulic acid using strain Streptomyces sp. V-1.

### Examples

### Example 1: Isolation of Streptomyces sp. V-1

Soil sample (5 g) taken from orchard is dissolved in 100 ml saline. 10 ml of this well-mixed solution is inoculumed to 50 ml GY biotransformation medium and incubated for 24 h at 30°C, 120 rpm min-1. And then ferulic acid is added (1%, w/v) and incubated for 20 h. This process is repeated for 3 ∼ 5 times. The above culture is diluted for 1,000,000 times and spread on solid medium, incubated at 30°C for 20 h. The subsequent colony is inoculumed to 50 ml GY biotransformation medium. After 24 h cultivation, ferulic acid (1%, w/v) is added for biotransformation at 120 rpm min-1. At last a strain which has the best ability to degrade ferulic acid and accumulate vanillin is chosen and named as Streptomyces sp. V-1, which has been deposited in China Center for Type Culture Collection on July 12, 2006 with the number of CCTCC M 206065.

Aerial mycelium of the above strain is brilliant yellow and substrate mycelium is slight yellow. Substrate mycelium has no septum and fragment. Spores are elliptical and the surface is smooth. This strain can utilize glucose, xylose, fructose, sucrose. The strain can not utilize glutin and no hydrogen sulfide is produced. 16S rDNA sequence of Streptomyces sp. V-1 has a similarity of 97 ∼ 98% to those of many Streptomyces strains.

In the above method of isolation strains capable of transforming ferulic acid to vanillin, solid slant medium contains glucose 10 g I-1, yeast powder 5 g I-1, peptone 10 g I-1, malt extract 10 g I-1, sodium chloride 5 g I-1, agar 16 g I-1 (pH 7.2). The medium is sterilized at 115°C for 20 min.

In the above method of isolation strains capable of transforming ferulic acid to vanillin, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 2

Seed culture of strain Streptomyces sp. V-1 after 24 h incubation is inoculumed to GY biotransformation medium (6%, v/v). After incubated for 18 h, ferulic acid is added to a final concentration of 5 g I-1 for biotransformation. The biotransformation condition is 30°C, 120 rpm min-1. Samples are removed at 20 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 18 h.
4. Biotransformation: ferulic acid (5 g I-1) is added to the culture obtained from step 4 and cultivated at 30°C, 120 rpm min-1 for 20 h.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 1.5 g I-1, with a molar yield of 38.3%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium is sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 3

Seed culture of strain Streptomyces sp. V-1 after 24 h incubation is inoculumed to GY biotransformation medium (6%, v/v). After incubated for 28 h, ferulic acid is added to a final concentration of 5 g I-1 for biotransformation. The biotransformation condition is 30°C, 120 rpm min-1. Samples are removed at 20 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 28 h.
4. Biotransformation: ferulic acid (5 g I-1) is added to the culture obtained from step 4 and cultivated at 30°C, 120 rpm min-1 for 20 h.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 1.6 g I-1, with a molar yield of 40.8%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium was sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 4

Seed culture of strain Streptomyces sp. V-1 after 24 h incubation is inoculumed to GY biotransformation medium (6%, v/v). After incubated for 24 h, ferulic acid is added to a final concentration of 5 g I-1 for biotransformation. The biotransformation condition is 30°C, 120 rpm min-1. Samples are removed at 20 h and the concentrations of vanillin are analyzed.

The detailed steps of vanillin production from ferulic acid using strain Streptomyces sp. V-1 are as follows:
1. Cultivation of slants: strain Streptomyces sp. V-1 was inoculumed to slants and statically cultivated at 30°C for 24 h.
2. Preparation of seed culture: slants obtained from step 1 is inoculmed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 24 h.
4. Biotransformation: ferulic acid (5 g I-1) is added to the culture obtained from step 4 and cultivated at 30°C, 120 rpm min-1 for 20 h.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 2.0 g I-1, with a molar yield of 51.1%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium was sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 5

Seed culture of strain Streptomyces sp. V-1 after 24 h incubation is inoculumed to GY biotransformation medium (6%, v/v). After incubated for 24 h, ferulic acid is added to a final concentration of 10 g I-1 for biotransformation. The biotransformation condition is 30°C, 120 rpm min-1. Samples are removed at 20 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 24 h.
4. Biotransformation: ferulic acid (10 g I-1) is added to the culture obtained from step 4 and cultivated at 30°C, 120 rpm min-1 for 20 h.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 5.0 g I-1, with a molar yield of 63.8%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium is sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 6

Seed culture of strain Streptomyces sp. V-1 after 24 h incubation is inoculumed to GY biotransformation medium (6%, v/v). After incubated for 24 h, ferulic acid is added to a final concentration of 15 g I-1 for biotransformation. The biotransformation condition is 30°C, 120 rpm min-1. Samples are removed at 20 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 24 h.
4. Biotransformation: ferulic acid (15 g I-1) is added to the culture obtained from step 4 and cultivated at 30°C, 120 rpm min-1 for 20 h.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 6.5 g I-1, with a molar yield of 55.3%.

In the above method of vanillin production from ferulic acid by biotransformation using a Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium was sterilized at 115°C for 20 min. The solid slant medium is same as the liquid seed medium and supplied with 1.6% agar (weight/volume).

In the above method of vanillin production from ferulic acid by biotransformation using the Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 7

Seed culture of strain Streptomyces sp. V-1 after 24 h incubation is inoculumed to GY biotransformation medium (6%, v/v). After incubated for 24 h, ferulic acid is added to a final concentration of 10 g I-1 for biotransformation. The biotransformation condition is 30°C, 120 rpm min-1. Samples are removed at 17 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 24 h.
4. Biotransformation: ferulic acid (10 g I-1) is added to the culture obtained from step 4 and cultivated at 30°C, 120 rpm min-1 for 17 h.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 5.75 g I-1, with a molar yield of 73.4%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium was sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 8

Seed culture of strain Streptomyces sp. V-1 after 24 h incubation is inoculumed to GY biotransformation medium (6%, v/v). After incubated for 24 h, ferulic acid is added to a final concentration of 10 g I-1 for biotransformation. The biotransformation condition is 30°C, 120 rpm min-1. Samples are removed at 15 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 24 h.
4. Biotransformation: ferulic acid (10 g I-1) is added to the culture obtained from step 4 and cultivated at 30°C, 120 rpm min-1 for 15 h.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 4.6 g I-1, with a molar yield of 58.7%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium was sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 9

Seed culture of strain Streptomyces sp. V-1 after 24 h incubation is inoculumed to GY biotransformation medium (6%, v/v). After incubated for 24 h, ferulic acid is added to a final concentration of 10 g I-1 for biotransformation. The biotransformation condition is 30°C, 120 rpm min-1. After 12 h cultivation macroporous adsorbent resin DM11 (5%, wet weight/volume) is added. When the concentration of feulic acid is lower than 2 g I-1, an additional 5 g I-1 ferulic acid is added. The total concentration of ferulic acid is 45 g I-1. Samples are removed at 55 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: the slant obtained from step 1 is inoculumed to liquid seed medium and cultivated at 30°C, 120 rpm min-1 for 24 h.
3. Cultivation of biotransformation cells: the seed culture obtained from step 2 is inoculumed to GY biotransformation medium (6%, v/v) and cultivated at 30°C, 120 rpm min-1 for 24 h.
4. Biotransformation: ferulic acid (10 g I-1) is added to the culture obtained from step 4 and cultivated at 30°C, 120 rpm min-1. After 12 h cultivation macroporous adsorbent resin DM11 (5%, wet weight/volume) is added. When the concentration of feulic acid is lower than 2 g I-1, an additional 5 g I-1 ferulic acid is added. The total concentration of ferulic acid is 45 g I-1.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 16.5 g I-1, with a molar yield of 46.8%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium is sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 10

Seed culture of strain Streptomyces sp. V-1 after 24 h incubation is inoculumed to GY biotransformation medium (6%, v/v). After incubated for 24 h, ferulic acid is added to a final concentration of 10 g I-1 for biotransformation. The biotransformation condition is 30°C, 120 rpm min-1. After 12 h cultivation macroporous adsorbent resin DM11 (8%, wet weight/volume) is added. When the concentration of feulic acid is lower than 2 g I-1, an additional 5 g I-1 ferulic acid is added. The total concentration of ferulic acid is 45 g I-1. Samples are removed at 55 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 24 h.
4. Biotransformation: ferulic acid (10 g I-1) is added to the culture obtained from step 4 and cultivated at 30°C, 120 rpm min-1. After 12 h cultivation macroporous adsorbent resin DM11 (8%, wet weight/volume) is added. When the concentration of feulic acid is lower than 2 g I-1, an additional 5 g I-1 ferulic acid is added. The total concentration of ferulic acid is 45 g I-1.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 19.2 g I-1, with a molar yield of 54.5%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium is sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

### Example 11

Seed culture of strain Streptomyces sp. V-1 after 24 h incubation is inoculumed to GY biotransformation medium (6%, v/v). After incubated for 24 h, ferulic acid is added to a final concentration of 10 g I-1 for biotransformation. The biotransformation condition is 30°C, 120 rpm min-1. After 12 h cultivation macroporous adsorbent resin DM11 (10%, wet weight/volume) is added. When the concentration of feulic acid is lower than 2 g I-1, an additional 5 g I-1 ferulic acid is added. The total concentration of ferulic acid is 45 g I-1. Samples are removed at 55 h and the concentrations of vanillin are analyzed.

The detailed steps of vanillin production from ferulic acid using strain Streptomyces sp. V-1 are as follows:
1. Cultivation of slants: strain Streptomyces sp. V-1 is inoculumed to slants and statically cultivated at 30°C for 24 h.
2. Preparation of seed culture: slants obtained from step 1 is inoculumed to liquid seed medium and cultivated at 30°C, 120 rpm min-1 for 24 h.
3. Cultivation of biotransformation cells: seed culture obtained from step 2 is inoculumed to GY biotransformation medium (6%, v/v) and cultivated at 30°C, 120 rpm min-1 for 24 h.
4. Biotransformation: ferulic acid (10 g I-1) is added to the culture obtained from step 4 and cultivated at 30°C, 120 rpm min-1. After 12 h cultivation macroporous adsorbent resin DM11 (10%, wet weight/volume) is added. When the concentration of feulic acid is lower than 2 g I-1, an additional 5 g I-1 ferulic acid is added. The total concentration of ferulic acid is 45 g I-1.
5. Measurement on concentration of vanillin: biotransformation samples are extracted with butyl acetate (analytic grade). Concentrations of vanillin are analyzed by VARIAN 3380 gas chromatography. It is found that the concentration of vanillin is 17.5 g I-1, with a molar yield of 49.6%.

In the above method of vanillin production from ferulic acid by biotransformation using the Streptomyces sp. strain, the seed medium contains glucose 10 g I-1, yeast powder 5 g I-1, peptone 10 g I-1, malt extract 10 g I-1, sodium chloride 5 g I-1 (pH 7.2). The medium is sterilized at 115°C for 20 min. The solid slant medium is same as the liquid seed medium and supplied with 1.6% agar (weight/volume).

In the above method of vanillin production from ferulic acid by biotransformation using a Streptomyces sp. strain, the GY biotransformation medium contains glucose 15 g I-1, yeast powder 8 g I-1, magnesium sulfate 0.8 g I-1, sodium chloride 2 g I-1 (pH 7.2). The medium is sterilized at 115°C for 20 min.

## Claims

1. A Streptomyces strain, wherein the strain is named as Streptomyces sp. V-1 and has been deposited in China Center for Type Culture Collection on July 12, 2006 with the number of CCTCC M 206065. The characteristic of this strain is: aerial mycelium is brilliant yellow and substrate mycelium is slight yellow. Substrate mycelium has no septum and fragment. Spores are elliptical and the surface is smooth. The strain can utilize glucose, xylose, fructose, sucrose. The strain can not utilize glutin and no hydrogen sulfide is produced. Its 16S rDNA sequence has a similarity of 97∼98% to those of many Streptomyces strains.

2. The strain Streptomyces sp. V-1 CCTCC M 206065 according to claim 1, wherein the incubation steps are:
(1) Cultivation of slants: strain Streptomyces sp. V-1 is inoculumed to solid slants and statically cultivated at 30°C for 24 h.
(2) Preparation of seed culture: slants obtained from step 1 are inoculumed to liquid seed medium and cultivated at 30°C, 120 rpm min-1 for 24 h.

3. The characteristic of the strain Streptomyces sp. V-1 according to claim 2, wherein the seed medium contains glucose 10 g I-1, yeast powder 5 g I-1, peptone 10 g I-1, malt extract 10 g I-1, sodium chloride 5 g I-1 (pH 7.2). The above medium is sterilized at 115°C for 20 min. The solid slant medium is same as the liquid seed medium and supplied with 1.6% agar (weight/volume).

4. The characteristic of the uses of Streptomyces sp. V-1 in the vanillin production from ferulic acid by biotransformation according to claim 1 or 2, wherein GY biotransformation medium is used and the steps are:
(1) Cultivation of biotransformation cells: seed culture obtained from claim 2 is inoculumed to GY biotransformation medium (6%, v/v) and cultivated at 30°C, 120 rpm min-1 for 10 ∼ 30 h.
(2) Biotransformation: ferulic acid (5 ∼ 15 g I-1) is added to the culture obtained from step 1 and cultivated at 30°C, 120 rpm min-1 for 12 ∼ 24 h.

5. The characteristic of the uses of Streptomyces sp. V-1 in the vanillin production from ferulic acid by biotransformation according to claim 4, wherein in the fed-batch biotransformation of ferulic acid, macroporous adsorbent resin DM11 is added (2% ∼ 12%, wet weight/volume) after 12-h cultivation.

6. The characteristic of the uses of Streptomyces sp. V-1 in the vanillin production from ferulic acid by biotransformation according to claim 4, wherein when the concentration is below 2 g I-1, additional 5 g I-1 ferulic acid is added until the final concentration of ferulic acid reaches 45 g I-1 and the total biotransformation time is 55 h.

7. The characteristic of the uses of Streptomyces sp. V-1 in the vanillin production from ferulic acid by biotransformation according to claim 4, wherein the GY biotransformation medium contains glucose 15 g I-1, yeast powder 8 g I-1, magnesium sulfate 0.8 g I-1, sodium chloride 2 g I-1 (pH 7.2).

8. The characteristic of the uses of Streptomyces sp. V-1 in the vanillin production from ferulic acid by biotransformation according to claim 4, wherein when 5 ∼ 10% (wet weight/volume) of macroporous adsorbent resin DM11 is added in the fed-batch biotransformation.
